# EUROPÄISCHE PATENTSCHRIFT

(11) **EP 0 415 198 B1**
(45) Veröffentlichungstag und Bekanntmachung des Hinweises auf die Patenterteilung: **25.01.1995**
(21) Anmeldenummer: 90115745.3
(22) Anmeldetag: 17.08.1990
(51) Int. Cl.: C07H 15/04, A61K 31/70

(54) **Ketophosphamidglycoside, Verfahren zu ihrer Herstellung sowie ihre Verwendung als pharmazeutische Wirkstoffe**
Ketophosphamidyl glycosides, process for their preparation and their use as pharmaceutical agents
Glycosides ketophosphamidyls, procédé pour leur préparation et leur utilisation comme agents pharmaceutiques

(30) Priorität: 30.08.1989 DE 3928659
(43) Veröffentlichungstag der Anmeldung: 06.03.1991
(73) Patentinhaber: Tietze, Lutz F., Prof. Dr., D-37077 Göttingen (DE)
(72) Erfinder: Tietze, Lutz-F., Dr., D-3400 Göttingen (DE); Fischer, Roland, Dr., D-6430 Bad Hersfeld (DE); Beller, Matthias, Dr., Cambridge, Massachusetts 02139 (US)

(56) Entgegenhaltungen:
- EP-A- 0 345 583
- US-A- 4 841 085
- LIEBIGS ANNALEN DER CHEMIE, Band 1990, Nr. 2, Februar 1990, Seiten 151-157, Weinheim, DE; L.F. TIETZE et al.: "Novel stable bioactivated cyclophosphamide derivatives for selective cancer chemotherapy, synthesis of aldophosphamide acetal-glycosides and their glycoconjugates"
- ANGEWANDTE CHEMIE INTERNATIONAL EDITION IN ENGLISH, Band 29, Nr. 7, Juli 1990, Seiten 782-783, Weinheim, DE; L.F. TIETZE et al.: "Development of tailor-made cytostatics activable by acid-catalyzed hydrolysis for selective tumor therapy"
- CARBOHYDRATE RESEARCH, Band 194, Dezember 1989, Seiten 155-162, Amsterdam, NL; L.F. TIETZE et al.: "Stereoselective synthesis of 1,1-dialkyl-1-methoxy-methyl glucosides (acetal-glucosides)"
- CARBOHYDRATE RESEARCH, Band 180, 1988, Seiten 253-262, Amsterdam, NL; L.F. TIETZE et al.: Stereoselective synthesis of (1-alkoxyalkyl) alpha- and beta-D-glucopyranosiduronates (acetal-glucopyranosiduronates): A new approach to specific cytostatics for the treatment of cancer"

## Beschreibung

Die Erfindung betrifft neue säurelabile Ketalglycoside von Aldophosphamid, ein Verfahren zu ihrer Herstellung und ihre Verwendung als hochselektive Cytostatika in der Krebstherapie.

Es ist bekannt, daß maligne Zellen eine gegenüber dem Normalgewebe erhöhte Glycolyse und damit Lactatproduktion aufzeigen und der pH-Wert im Tumorgewebe durch intravenös verabreichte Glucose gesenkt werden kann (vgl. S. Tanneberger, Experimentelle und klinische Tumorchemotherapie; Allgemeine Tumorchemotherapie; G. Fischer Verlag, Stuttgart/New York 1980; Naturwiss. 46, 2(1959); Cancer Res. 42, 1498 (1982); 42, 1505 (1982)).

In der Vergangenheit wurde versucht, diese Unterschiede im pH-Wert zwischen normalem und Tumorgewebe für eine selektive Tumortherapie auszunutzen (vergl. Liebigs Ann. Chem. 1987, 847-856; Tetrahedron Lett. 22 (1981) 239-242). Es war angestrebt worden, alkylierende Verbindungen, die wegen einer zu geringen Differenzierung zwischen gesundem und malignem Gewebe eine sehr geringe therapeutische Breite aufweisen, in untoxische, säurelabile Prodrug-Formen zu überführen, die nur im Tumorgewebe aufgrund des dort vorherrschenden erniedrigten pH-Wert selektiv gespalten werden zum aktiven, alkylierend wirkenden Cytostatikum. Auf diese Weise sollte eine selektive Tumortherapie angestrebt werden.

Antitumor Aldophosphamide sind in US.A. 4841085 beschrieben.

Es hatte sich aber gezeigt, daß die in der oben angeführten Literaturstelle hergestellten Verbindungen sich nicht als so säurelabil erwiesen, als daß sie selektiv im Tumorgewebe zum aktiven cytociden Agenz rückgespalten wurden.

Es wurde nun überraschenderweise festgestellt, daß die folgenden genannten Verbindungen untoxisch sind, während sie bei dem in Tumorgewebe durch Hyperglykämie erreichbaren pH-Wert durch Hydrolyse in cytocide Verbindungen überführt werden können.

Die neuen Verbindungen entsprechen der allgemeinen Formel (I), 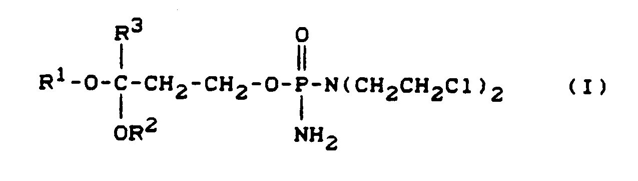
in welcher
- R¹ -: für einen Pyranosyl-Rest der Formel 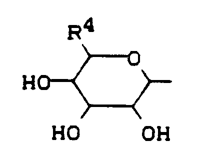 in der D- oder L-Form steht, worin
- R⁴ -: die Gruppe der Formel -CH₂OH oder geradkettiges oder verzweigtes Alkyl mit bis zu 4 Kohlenstoffatomen bedeutet,
wobei die OH-Funktionen des Zuckers gegebenenfalls geschützt sind,
- R² -: für geradkettiges oder verzweigtes Alkyl mit bis zu 8 Kohlenstoffatomen steht
und
- R³ -: für geradkettiges oder verzweigtes Alkyl mit bis zu 10 Kohlenstoffatomen steht.

Bevorzugt sind Verbindungen der allgemeinen Formel (I), in welcher
- R¹ -: für einen Hexopyranosyl-Rest der Formel 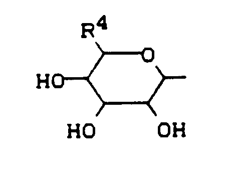 in der D- oder L-Form steht,
worin
- R⁴ -: die Gruppe der Formel -CH₂OH oder Methyl bedeutet,
wobei die OH-Funktionen des Zuckers gegebenenfalls geschützt sind,
- R² -: für geradkettiges oder verzweigtes Alkyl mit bis zu 6 Kohlenstoffatomen steht
und
- R³ -: für geradkettiges oder verzweigtes Alkyl mit bis zu 8 Kohlenstoffatomen steht.

Besonders bevorzugt sind Verbindungen der allgemeinen Formel (I), in welcher
- R¹ -: für den D-Glucopyranosyl-Rest steht, wobei die OH-Funktionen des Zuckers gegebenenfalls geschützt sind,
- R² -: für geradkettiges oder verzweigtes Alkyl mit bis zu 4 Kohlenstoffatomen steht
und
- R³ -: für geradkettiges oder verzweigtes Alkyl mit bis zu 6 Kohlenstoffatomen steht.

Als OH-Schutzgruppen eignen sich die üblichen Schutzgruppen wie Methylbenzoyl, Benzoyl, Acetyl oder Benzyl. Bevorzugt sind Benzyl, Acetyl oder Methylbenzoyl. Besonders bevorzugt ist Acetyl (Ac).

Außerdem wurde ein Verfahren zur Herstellung der erfindungsgemäßen Verbindungen der allgemeinen Formel (I), gefunden,
das dadurch gekennzeichnet ist, daß man
Verbindungen der allgemeinen Formel (II) 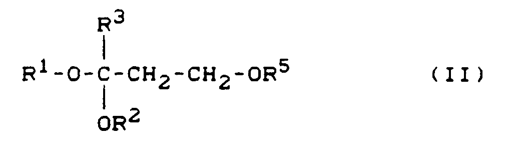
in welcher
R¹, R² und R³ die oben angegebene Bedeutung haben
und
- R⁵ -: für eine typische Hydroxyschutzgruppe steht,
zunächst unter Abspaltung der Schutzgruppe nach bekannter Methode in die Verbindungen der allgemeinen Formel (III) 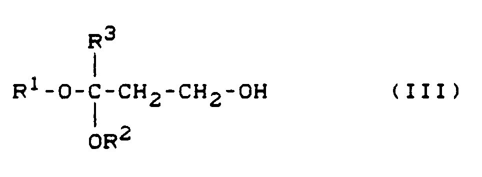
in welcher
R¹, R² und R³ die oben angegebene Bedeutung haben,
überführt, anschließend mit N,N-Bis(2-chlorethyl)-phosphorsäureamid-dichlorid der Formel (IV)

Cl₂PON(CH₂CH₂Cl)₂ (IV)

in inerten Lösemitteln, in Anwesenheit einer Base umsetzt, nachfolgend eine Ammonolyse durchführt und gegebenenfalls die Schutzgruppen am Zuckerrest abspaltet.

Das erfindungsgemäße Verfahren kann durch folgendes Formelschema erläutert werden: 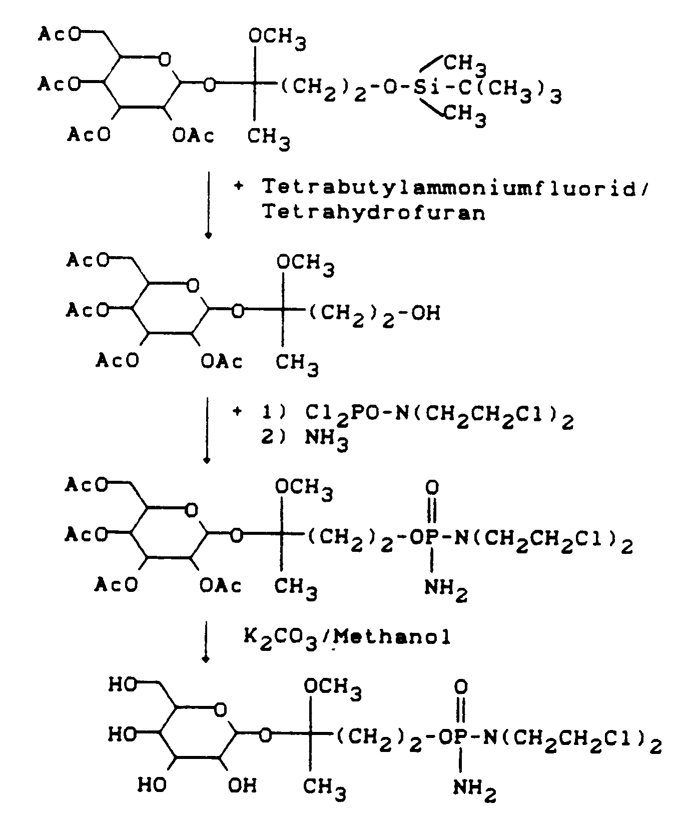
Hydroxyschutzgruppe im Rahmen der oben angegebenen Definition R⁵ steht im allgemeinen für Trimethylsilyl, Triethylsilyl, Triisopropylsilyl, tert.Butyl-dimethylsilyl, tert.Butyl-diphenylsilyl, Benzyl 2-Nitrobenzyl, Acetyl(Ac) oder Trifluormethoxy. Bevorzugt sind tert.-Butyl-dimethylsilyl, tert.Butyl-diphenylsilyl und Acetyl.

Die Abspaltung der Schutzgruppe erfolgt in an sich bekannter Weise, beispielsweise im Fall der Silylgruppen durch Tetrabutylammoniumfluorid oder durch hydrogenolytische Spaltung, wenn R⁵ für die Benzylgruppe steht, in Anwesenheit eines Katalysators mit Wasserstoff, in einem inerten Lösemittel [vgl. außerdem Th. Greene: "Protective Groups in Organic Synthesis", J. Wiley & Sons, 1981, New York].

Als Lösemittel sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylmin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Tetrahydrofuran und Dichlormethan.

Für die Umsetzung mit der Verbindung der Formel (IV) eignen sich die üblichen inerten organischen Lösemittel, die sich unter den Reaktionsbedingungen nicht verändern. Hierzu gehören bevorzugt Benzol, Toluol, Xylol, Dichlormethan oder Dimethylformamid. Ebenso ist es möglich, Gemische der genannten Lösemittel zu verwenden. Besonders bevorzugt ist Dichlormethan.

Als Basen eignen sich Natriumhydrid, Kaliumhydrid, Imidazol, Tetrazol und die üblichen organischen Amine. Hierzu gehören bevorzugt Alkylamine wie Triethylamin, Diisopropylamin, Dicyclohexylamin und Ethyldiisopropylamin. Besonders bevorzugt ist Triethylamin.

Die Basen werden im allgemeinen in einer Menge von 1 bis 5 Mol, bevorzugt von 1 bis 3 Mol, bezogen auf die Verbindungen der Formel (IV) eingesetzt.

Die Umsetzung wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Das erfindungsgemäße Verfahren wird im allgemeinen in einem Temperaturbereich von 0°C bis +6°C, bevorzugt bei Raumtemperatur durchgeführt.

Die oben aufgeführten Reaktionsbedingungen werden auch während der Ammonolyse nicht verändert. Das durch die Durchleitung des Ammoniakstroms verdampfte Lösemittel wird während der Umsetzung ersetzt.

Die Abspaltung der Schutzgruppen am Zuckerrest erfolgt nach üblicher Methode in inerten Lösemitteln in Anwesenheit einer Base oder durch Hydrogenolyse.

Als Basen eignen sich für die Abspaltung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kaliumethanolat, Kaliummethanolat oder Kalium-tert.butanolat. Besonders bevorzugt werden Natriumcarbonat oder Kaliumcarbonat eingesetzt.

Als Lösemittel eignen sich für die Abspaltung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Abspaltung wird im allgemeinen in einem Temperaturbereich von 0°C bis +100°C, bevorzugt von +20°C bis +80°C durchgeführt.

Im allgemeinen wird die Abspaltung bei Normaldruck durchgeführt. Es ist aber auch möglich, bei Unterdruck oder bei Überdruck zu arbeiten (z.B. von 0,5 bis 5 bar).

N,N-Bis(2-chlorethyl)-phosphorsäureamid-dichlorid der Formel (IV) ist bekannt [vgl. J. Am. Chem. Soc. 76, 655 (1954)].

Die Verbindungen der allgemeinen Formel (II) sind neu und können hergestellt werden, indem man
Verbindungen der allgemeinen Formel (V) 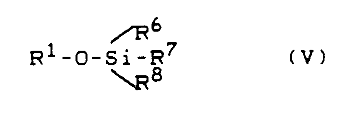
in welcher
R¹ die oben angegebene Bedeutung hat
und
- R⁶, R⁷ und R⁸: gleich oder verschieden sind und für C₁-C₈-Alkyl oder Phenyl stehen,
zunächst mit Verbindungen der allgemeinen Formel (VI) 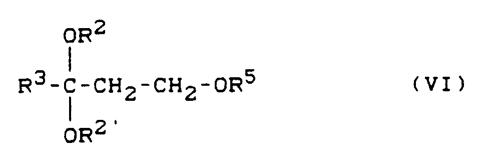
in welcher
R², R³ und R⁵ die oben angegebene Bedeutung haben
und
- R²′: die oben angegebene Bedeutung von R² hat und mit dieser gleich oder verschieden ist
umsetzt
und anschließend mit dem der Verbindung (VI) zugrunde liegenden Keton der allgemeinen Formel (VII)

R³-CO-(CH₂)₂-OR⁵ (VII)

in welcher
R³ und R⁵ die oben angegebene Bedeutung haben,
in inerten Lösemitteln, gegebenenfalls in Anwesenheit eines Katalysators umsetzt.

Als Lösemittel eignen sich die oben aufgeführten inerten Lösemittel. Bevorzugt ist Dichlormethan.

Als Katalysatoren eignen sich Lewis- und Brönstedsäuren wie Trifluoressigsäure, p-Toluolsulfonsäure, Camphersulfonsäure, Trifluormethansulfonsäuretrimethylsilylester und Jodtrimethylsilan. Im allgemeinen setzt man 0,01 bis 1, bevorzugt 0,05 bis 0,5 mol Katalysator, bezogen auf 1 mol Reaktionspartner ein.

Die Verbindungen der allgemeinen Formel (VII) werden im allgemeinen in einer Menge von 1 bis 10 mol, bevorzugt mit 3 mol, bezogen auf 1 mol der Verbindungen der allgemeinen Formeln (V) und (VI) eingesetzt.

Das Verfahren erfolgt im allgemeinen in einem Temperaturbereich von -78°C bis +2°C, bevorzugt bei -78°C.

Die Verbindungen der allgemeinen Formel (V) sind bekannt [vgl. W.A. Bonner, J. Org. Chem. 26, 908 (1961); L. Vargha und J. Kurzmann, Chem. Ber. 96, 2016 (1963)].

Die Verbindungen der allgemeinen Formeln (VI) und (VII) sind ebenfalls bekannt oder können nach üblicher Methode hergestellt werden [vgl. J. Org. Chem. 26, 908, 1961 und Can. J. Chem. 56, 2889, 1978, Heterocycles 15, 999 (1981); Chem. Pharm. Bull. 15, 1893 (1967)].

Die erfindungsgemäßen Verbindungen zeigen ein nicht vorhersehbares, wertvolles pharmakologisches Wirkspektrum.

Die säurelabilen Glycoside des inaktivierten Cyclophosphamids der allgemeinen Formel (I) stellen erstmalig ein Cytostatikum dar, das in seiner Transportform untoxisch ist. Bei dem im Tumorgewebe durch Hyperglykämie erreichbaren pH-Wert setzen sie durch Hydrolyse eine cytocide Verbindung, die Friedman-Säure frei.

Die zu spaltende Verbindung (Beispiel 5) wurde in 1 ml Wasser (bidest.) gelöst. Je 200 µl wurden in 1 ml eines Puffersystems gegeben, das auf 37°C thermostatiert war. Bei folgenden pH-Werten wurden Versuche durchgeführt: 5.0, 5.5, 6.0, 6.5, 7.0. Die Spaltungen wurden nach 1h, 2h, 4h, 8h, 24h und danach im Abstand von 24h mit NMR-Spektroskopie untersucht. Die Konzentrationen der benutzten Phosphatpuffersysteme betrug 0,05 mol/l.

Spaltungsversuche, die nach der oben aufgeführten Arbeitsvorschrift durchgeführt wurden, zeigen, daß bevorzugt die Verbindung aus Beispiel 5 bei pH = 6 mit einer Halbwertszeit von 4h gespalten wird.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche öle (z.B. Erdnuß/Sesamöl), Alkohole, (z.B. Ethylalkohol, Glycerin), Trägerstoffe, wie z.B. natürliche Gesteinsmehle (z.B. Kaoline, Tonerden, Talkum, Kreide), synthetische Gesteinsmehle (z.B hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether), Dispergiermittel (z.B. Lignin-Sulfitablaugen, Methylcellulose, Stärke und Polyvinylpyrrolidon) und Gleitmittel (z.B. Magnesiumstearat, Talkum, Stearinsäure und Natriumsulfat).

Die Applikation erfolgt in üblicher Weise, vorzugsweise oral oder parenteral, insbesondere perlingual oder intravenös Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitvervendet werden. Im Falle wäßriger Suspensionen können die Wirkstoffe außer den oben genannten Hilfsstoffen mit verschiedenen Geschmacksaufbesserern oder Farbstoffen versetzt werden.

Bei oraler Applikation sind solche galenischen Zubereitungen zu verwenden bei denen die Freisetzung der Wirkstoffe erst im Darm erfolgt.

Eine Freisetzung im Magen kann zu einer unerwünschten vorzeitigen Acidolyse der erfindungsgemäßen Substanzen führen.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Aplikation beträgt die Dosierung etwa 0,01 bis 20 mg/kg vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art der Applikation, aber auch aufgrund der Erkrankung und deren individuellem Verhalten gegenüber dem Medikament bzw. deren Art von dessen Formulierung und dem Zeitpunkt bzw. Intervall zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen.

Die Applikation von Verbindungen der allgemeinen Formel I mit R' für einen D-Glucopyranosyl-Rest an Kulturen von Mammcarcinom-Zellen ergab eine sehr geringe Toxizität bei pH 7.4 während die Überlebensrate der Krebszellen bei pH 6.2 um den Faktor 5 X 10⁻⁵ erniedrigt wurde.

Application von 500 mg/kg der obengenannten Verbindung an Sprague Dowly-Ratten, die einen transplantierten AH-13r Tumor trugen, führte bei einmaliger Gabe zu einer vollständigen Remission des Tumors.

### Ausgangsverbindungen

### Beispiel I

### 4-tert.Butyldiphenylsiloxybutan-2-on

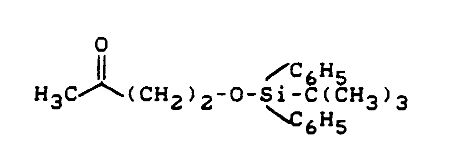

7,43 g (109 mmol) Imidazol und 20 g (72,8 mmol) tert.-Butyldiphenylsilylchlorid wurden in 200 ml Dichlormethan gelöst und auf 0°C abgekühlt. Zu dieser Lösung tropfte man 7,69 g (87,4 mmol) 4-Hydroxybutan-2-on. Nach Erwärmen auf Raumtemperatur wurde 12 Stunden (DC-Kontrolle) gerührt. Das Reaktiongsgemisch wurde zweimal mit 80 ml Eiswasser ausgeschüttelt, die organische Phase mit Natriumsulfat getrocknet und nach Evaporieren des Lösemittels im Ölpumpenvakuum destilliert. Man erhielt 22,3 g (68,4 mmol) Produkte.
Ausbeute: 94% der Theorie
R_{f} = 0,29 (tert.Butylmethylether/Petrolether 1:8)
Kp = 127-130/0.06 Torr
Schmp. = 38-40°C

| C₂₀H₂₆O₂S;(326.51) | | |
|---|---|---|
| Ber.: | C 73,57 | H 8,03 |
| Gef.: | C 73,59 | H 8,16 |

### Beispiel II

### 4-tert.Butyldimethylsiloxybutan-2-on

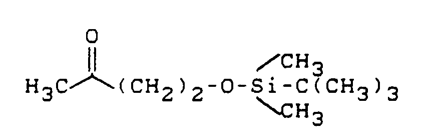

Die Verbindung wurde in Analogie zur Vorschrift des Beispiels I hergestellt.
Ausbeute: 92%. der Theorie
R_{f} = 0,34 (tert.Butylmethylether/Petrolether 1:8)
Kp = 63°C/15 Torr

| C₁₀H₂₂O₂Si (202,37) | | |
|---|---|---|
| Ber.: | C 59,35 | H 10,96 |
| Gef.: | C 59,32 | H 10,80 |

### Beispiel III

### 4-(tert.Butyldiphenylsiloxy)-2,2-dimethoxybutan

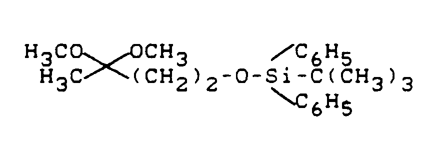

Die Ketalisierung wurde entsprechend E.G. Taylor, L.S. Chiang, Synthesis, 1977, 457 durchgeführt
Ausbeute: 78% der Theorie
Schmp.: 28-30°C
R_{f} = 0,44 (tert.Butylmethylether/Petrolether 1:8)
Kp: 145-150°C/0,05 Torr

| C₂₂H₃₂O₃Si (372,58) | | |
|---|---|---|
| Ber.: | C 70,92 | H 8,66 |
| Gef.: | C 70,96 | H 8,65 |

### Beispiel IV

### 4-(tert.Butyldimethylsiloxy-2,2-dimethoxybutan)

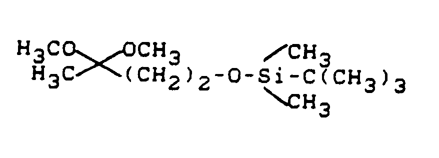

Die Titelverbindung wurde in Analogie zur Vorschrift des Beispiels III hergestellt.
Ausbeute: 86% der Theorie
R_{f} = 0,48 (tert.Butylmethylether/Petrolether 1:8)
Kp = 75°C/15 Torr

| C₁₂H₂₈O₃Si (248,43) | | |
|---|---|---|
| Ber.: | C 58,02 | H 11,36 |
| Gef.: | C 58,18 | H 11,30 |

### Herstellungsbeispiele

### Beispiel 1

### (1′RS)-3′-Diphenyl-tert.butylsiloxy-1′-methoxy-1′-methylpropyl-2,3,4,6-tetra-O-acetyl-β-D-glucopyranosid

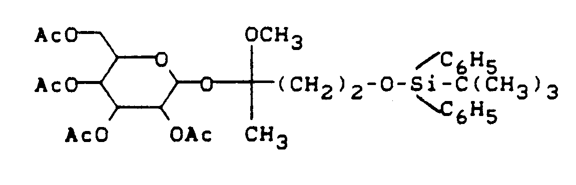

420 mg (1,00 mmol) 1-O-Trimethylsilyl-2,3,4,6-tetra-O-acetyl-β-D-glucopyranosid wurden in 15-20 ml Dichlormethan gelöst und unter Feuchtigkeitsausschluß und Schutzgas (Argon) mit 980 mg (3,00 mmol) der Verbindung aus Beispiel I und 373 mg (1,00 mmol) der Verbindung aus Beispiel III versetzt. Nach Abkühlen auf -78°C addierte man 20 mol% Trifluormethansulfonsäuretrimethylsilylester (TMS-Triflat) und rührte bis zum vollständigen Umsatz (DC-Kontrolle) bei dieser Temperatur. Durch Zugabe von 1 ml einer Mischung aus Triethylamin und Ethanol (1:1) wurde die Reaktion beendet. Anschließende schnelle Säulenfiltration der kalten Lösung über ca. 5 g Kieselgel (Elutionsmittel tert.Butylmethylether) und Evaporieren des Lösemittels im Vakuum lieferten das Rohprodukt. Die weitere Reinigung erfolgte durch Flashchromatographie an 50 g Kieselgel (Elutionsmittel: tert.Butylmethylether/Petrolether/Triethylamin 100:100:0,7). Nach dem Einengen der gesammelten Fraktionen wurde die Substanz bei Raumtemperatur im Hochvakuum getrocknet und bis zur weiteren Verwendung unter Schutzgas bei -20°C gelagert. Man erhielt 269 mg (0,39 mmol) der Titelverbindung als farbloses Öl.
Ausbeute: 39% der Theorie
R_{f} 0,41 (tert.Butylether/Petrolether 2:1)

| C₃₅H₄₈O₁₂Si (688,8) | | |
|---|---|---|
| Ber.: | C 61,03 | H 7,02 |
| Gef.: | C 61,06 | H 7,11 |

### Beispiel 2

### (1′R,S)-3′-Dimethyl-tert.butylsiloxy-1′-methoxy-1′-methylpropyl-2,3,4,6-tetra-O-acetyl-β-D-glucopyranosid

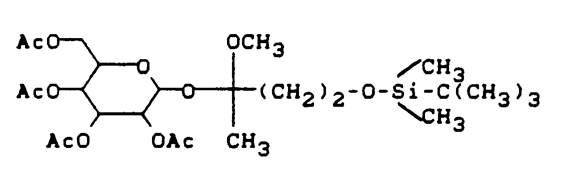

Die Titelverbindung wurde in Analogie zur Vorschrift des
Beispiels 1 hergestellt.
husbeute: 28% der Theorie
R_{f} = 0,48 (Essigester/Petrolether 1:1)

| C₂₅H₁₄O₁₂Si (564,70) | | |
|---|---|---|
| Ber.: | C 53,17 | H 7,85 |
| Gef.: | C 53,51 | H 8,01 |

### Beispiel 3

### (1′R,S)-3′-Hydroxy-1′-methoxy-1′-methylpropyl-2,3,4,6-tetra-O-acetyl-β-D-glucopyranosid

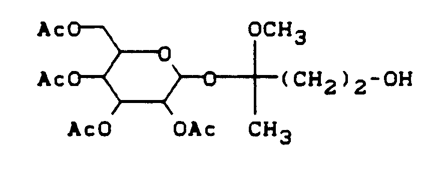

269 mg (0,39 mmol) der Verbindung aus Beispiel 1 wurden in 5 ml Tetrahydrofuran gelöst und bei Raumtemperatur mit einer Suspension von 96 mg Tetrabutylammoniumfluorid x 3 H₂O (vor der Reaktion 2 Stunden im Hochvakuum bei Raumtemperatur getrocknet) in 5 ml Tetrahydrofuran versetzt. Die Reaktionsmischung rührte bei 4°C bis zum vollständigen Umsatz (DG-Kontrolle, Reaktionszeit ca. 4-6 Stunden). Dann wurde das Lösemittel im Vakuum entfernt und das Rohprodukt an 25 g Kieselgel (32-63 µm, Elutionsmittel: Dichlormethan/Petrolether/Ethanol 15.5:1 mit 0,7% Triethylamin) flashchromatographiert. Man erhält 165 mg (0,37 mmol) eines farblosen Öls.
Ausbeute: 94% der Theorie
R_{f} = 0,27 (Dichlormethan/Petrolether/Ethanol 20:5:1)

| C₁₉H₃₀O₁₂ (450,4) | | |
|---|---|---|
| Ber.: | C 50,66 | H 6,71 |
| Gef.: | C 50,59 | H 6,76 |

### Beispiel 4

### (3 RS, RS-P)-[3-Methoxy-3-(2,3,4,6-tetra-O-acetyl-β-D-glucopyranosyloxy)butyl]-N,N-bis-(2-chlorethyl)phosphorsäurediamidat

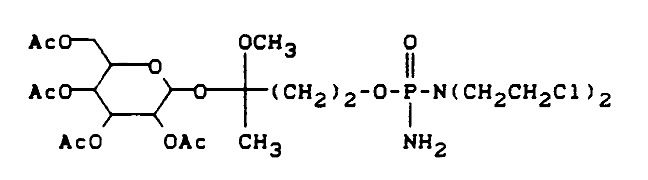

490 mg (1,09 mmol) der Verbindung aus Beispiel 3 wurden in 15 ml Dichlormethan unter Feuchtigkeitsausschluß und Schutzgas gelöst und mit 3 ml Triethylamin sowie 1,13 g (4,35 mmol) Bis-(2-chlorethyl)-phosphorsäureamiddichlorid versetzt. Die Mischung rührte 36 Stunden bei Raumtemperatur (DC-Kontrolle). Dann leitete man 1,5 Stunden einen kräftigen trockenen Ammoniakstrom durch die Lösung (verdampftes Lösemittel wird ersetzt). Der entstandene Niederschlag wird durch Flashsäulenfiltration über ca. 10 g Kieselgel (Dichlormethan/Petrolether/Ethanol 20:5:1 mit 0,7% Triethylamin) abgetrennt. Nach Evaporieren des Lösemittels im Vakuum wird das Rohprodukt durch Flashchromatographie an 50 g Kieselgel (32-63 µm, Dichlormethan/Petrolether/Ethanol 20:5:1 mit 0,7% Triethylamin) gereinigt. Es verbleiben 501 mg (0,81 mmol) eines leicht gelben kristallinen Schaums.
Ausbeute: 74% der Theorie
R_{f} = 0,24 (Dichlormethan/Petrolether/Ethanol 3:1:1)

| C₂₃H₃₉Cl₂N₂O₁₃P (653,44) | | | |
|---|---|---|---|
| Ber.: | C 42,28 | H 6,04 | N 4,29 |
| Gef.: | C 42,69 | H 6,22 | N 4,76 |

### Beispiel 5

### (3 RS, RS-P) 3-Methoxy-3-(β-D-glucopyranosyloxy)butyl]-N,N-bis-(2-chlorethyl)-phosphorsäurediamidat

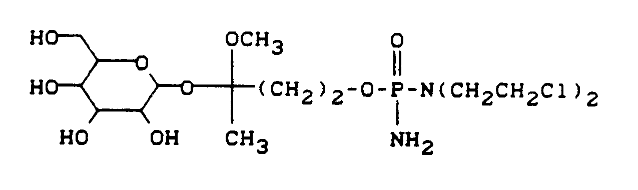

Zu einer Lösung von 501 mg (0,81 mmol) peracetyliertem Ketophosphamidacetalglucosids (4) in 5 ml Methanol wurden 25 mg aktiviertes Kaliumcarboant addiert. Die Mischung rührte ca. 10 Minuten (DC-Kontrolle); dann verdünnte man mit 10 ml tert.Butylmethylether und filtrierte die Lösung schnell über 10-15 g Celite. Nach dem Entfernen des Lösemittels im Vakuum verbleiben 349 mg (0,72 mmol) eines gelben kristallinen Schaums.
Ausbeute: 89% der Theorie
R_{f} = 0,30 (Methylenchlorid/Petrolether/Ethanol 3:1:1)

| C₁₅H₃₁Cl₂N₂O₉P (485,30) | | | |
|---|---|---|---|
| Ber.: | C 37,12 | H 6,44 | N 5,77 |
| Gef.: | C 37,35 | H 6,42 | N 5,94 |

## Patentansprüche

1. Verbindungen der allgemeinen Formel (I), 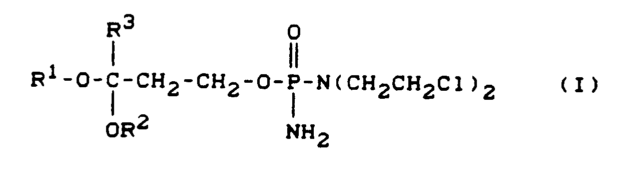 in welcher
R¹ - für einen Pyranosyl-Rest der Formel 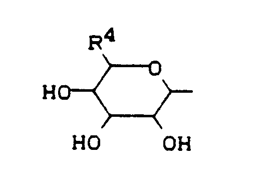 in der D- oder L-Form steht,
worin
R⁴ - die Gruppe der Formel -CH₂OH oder geradkettiges oder verzweigtes Alkyl mit bis zu 4 Kohlenstoffatomen bedeutet,
wobei die OH-Funktionen des Zuckers gegebenenfalls geschützt sind,
R² - für geradkettiges oder verzweigtes Alkyl mit bis zu 8 Kohlenstoffatomen steht
und
R³ - für geradkettiges oder verzweigtes Alkyl mit bis zu 10 Kohlenstoffatomen steht.

2. Verbindungen der allgemeinen Formel (I), gemäß Anspruch 1,
in welcher
R¹ - für einen Hexopyranosyl-Rest der Formel 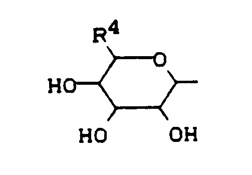 in der D- oder L-Form steht,
worin
R⁴ - die Gruppe der Formel -CH₂OH oder Methyl bedeutet,
wobei die OH-Funktionen des Zuckers gegebenenfalls geschützt sind,
R² - für geradkettiges oder verzweigtes Alkyl mit bis zu 6 Kohlenstoffatomen steht
und
R³ - für geradkettiges oder verzweigtes Alkyl mit bis zu 8 Kohlenstoffatomen steht.

3. Verbindungen der allgemeinen Formel (I), gemäß Anspruch 1
in welcher
R¹ - für den D-Glucopyranosyl-Rest steht,
wobei die OH-Funktionen des Zuckers gegebenenfalls geschützt sind,
R² - für geradkettiges oder verzweigtes Alkyl mit bis zu 4 Kohlenstoffatomen steht
und
R³ - für geradkettiges oder verzweigtes Alkyl mit bis zu 6 Kohlenstoffatomen steht.

4. Verfahren zur Herstellung der Verbindungen der allgemeinen Formel (I), gemäß Anspruch 1 das dadurch gekennzeichnet ist, daß man Verbindungen der allgemeinen Formel (II) 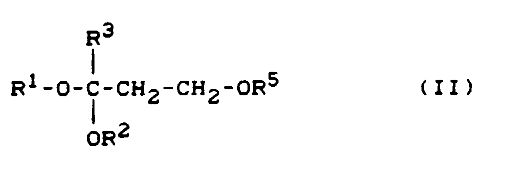 in welcher
R¹, R² und R³ die oben angegebene Bedeutung haben
und
R⁵ - für eine typische Hydroxyschutzgruppe steht,
zunächst unter Abspaltung der Schutzgruppe nach bekannter Methode in die Verbindungen der allgemeinen Formel (III) 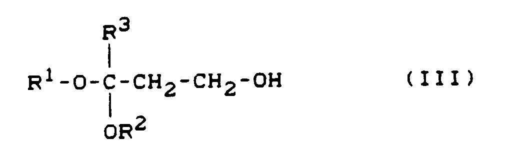 in welcher
R¹, R² und R³ die oben angegebene Bedeutung haben,
überführt, anschließend mit N,N-Bis(2-chlorethyl)phosphorsäureamid-dichlorid der Formel (IV)
Cl₂PON(CH₂CH₂Cl)₂ (IV)
in inerten Lösemitteln, in Anwesenheit einer Base umsetzt, nachfolgend eine Ammonolyse durchführt und gegebenenfalls die Schutzgruppen am Zuckerrest abspaltet.

5. Arzneimittel enthaltend eine der Verbindungen gemäß den Ansprüchen 1 bis 3.

## Claims

1. Compounds of general formula (I), 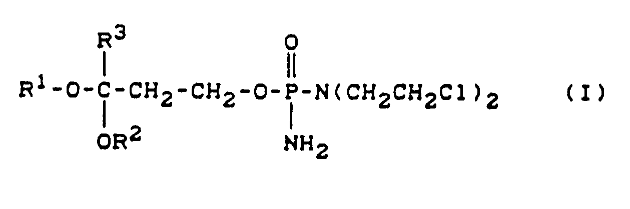 in which
R¹ - represents a pyranosyl-moiety of the formula 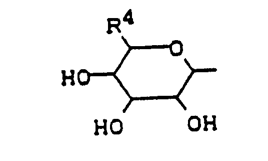 of the **D-** or **L-**Form,
wherein
R⁴ - the group of the formula means -CH₂OH or straight-chain or branched alkyl with up to 4 carbon atoms
whereby the OH-functions of the sugar are protected when applicable,
R² - represents a straight-chain or branched alkyl with up to 8 carbon atoms
and
R³ - represents a straight-chain or branched alkyl with up to 10 carbon atoms.

2. Compounds of general formula (I), according to Claim 1
in which
R¹ - represents a hexopyranosyl-moiety of the formula 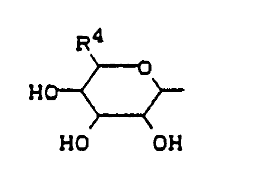 in the **D-** or L-Form,
wherein
R⁴ - the group of the formula means -CH₂OH or methyl,
whereby the OH-functions of the sugar are protected when applicable,
R² - represents a straight-chain or branched alkyl with up to 6 carbon atoms
and
R³ - represents a straight-chain or branched alkyl with up to 8 carbon atoms.

3. Compounds of general formula (I), according to Claim 1
in which
R¹ - represents a glucopyranosyl-moiety
whereby the OH-functions of the sugar are protected when applicable,
R² - represents a straight-chain or branched alkyl with up to 4 carbon atoms
and
R³ - represents a straight-chain or branched alkyl with up to 6 carbon atoms.

4. Procedures for the production of the compounds of general Formula (I), according to Claim 1
that is identified, in that
compounds of general formula (II), 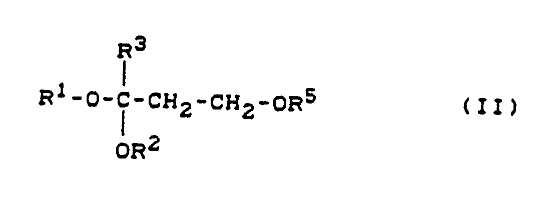 in which
R¹, R² and R³ have the above shown meaning
and
R⁵ - represents a typical hydroxy protecting group,
are transformed firstly by cleavage of the protecting groups, according to the known methods, into the compounds of general formula (III) 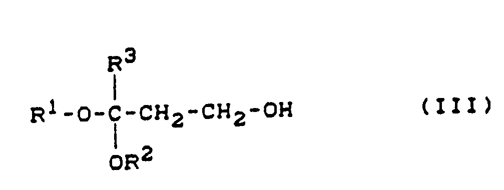 in which
R¹, R² and R³ have the above shown meaning,
subsequently converted with N,N-bis(2-chloroethyl)-phosphoric acid amide dichloride of formula (IV)
Cl₂PON(CH₂CH₂Cl)₂ (IV)
in an inert solvent in the presence of a base, thereinafter ammonolysis is carried out and when applicable cleavage of the protecting groups at the sugar moiety.

5. Drugs containing one of the compounds according to Claims 1 to 3

## Revendications

1. Composé avec la formule générale (I), 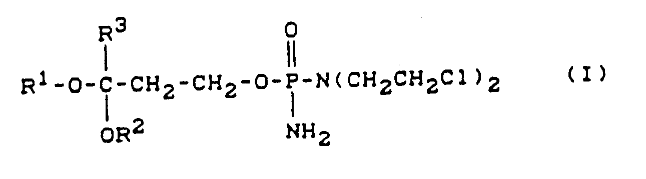 dans laquelle
R¹ - représente un reste pyranosyle avec la formule 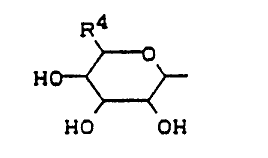 et apparait sous la forme D ou L,
dans lequel
R⁴- signifie le groupe avec la formule CH₂OH ou un alkyle de chaine droite ou de chaine ramifiée avec jusqu'à 4 carbones
et les fonctions OH du sucre dans certains cas sont protégées,
R² - représente un alkyle de chaine droite ou chaine ramifiée avec jusqu'à 8 carbones.
et
R³ - représente un alkyle de chaine droite ou chaine ramifiée avec jusqu'à 10 carbones.

2. Composés avec la formule générale (I), conforme à la révindication 1
dans laquelle
R¹ - représente un reste hexapyranosyle avec la formule 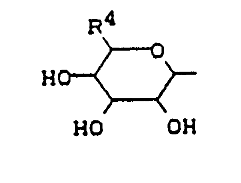 et apparait sous la forme D ou L,
dans lequel
R⁴- signifie le groupe avec la formule CH₂OH ou un groupe méthyle
et les fonctions OH du sucre dans certains cas sont protégées,
R² - représente un alkyle de chaine droite ou chaine ramifiée avec jusqu'à 6 carbones.
et
R³ - représente un alkyle de chaine droite ou chaine ramifiée avec jusqu'à 8 carbones.

3. Composé avec la formule générale conforme à la révindication 1
dans laquelle
R¹ - représente un reste D-glucopyranosyle,
dans lequel les fonctions OH du sucre dans certains cas sont protégées,
R² - représente un alkyle de chaine droite ou chaine ramifiée avec jusqu'à 4 carbones.
et
R³ - représente un alkyle de chaine droite ou chaine ramifiée avec jusqu'à 6 carbones.

4. Procédé pour la préparation des composés avec la formule générale (I) conforme à la révindication 1
Ce procéde ce caractérise par le fait que les composés avec la formule générale (II) 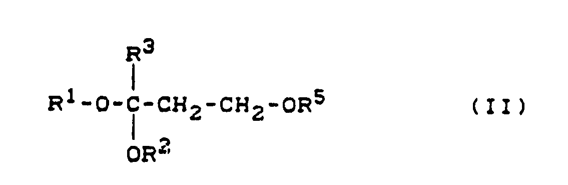 dans laquelle R¹, R² et R³ ont la signification indiquée çi dessus
et
R⁵ - représente un groupe de protection OH typique,
sont d'abord transformés par scission du groupe de protection par une méthode déjà connue en composés avec la formule générale (III) 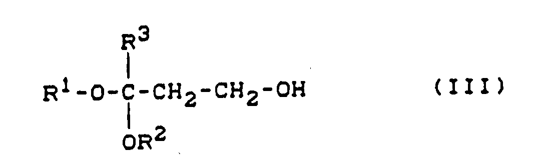 dans laquelle
R¹, R² et R³ ont la signification décrite çi dessus,
ensuite on fait réactionner avec N,N-bis(2-chloroéthyl)-amide de l'acide phosphorique-dichloride avec la formule (IV)
Cl₂PON(CH₂CH₂Cl)₂ (IV)
dans des solvants inertes, avec une base, ensuite on fait une ammonolyse est en certains cas on déprotége le sucre.

5. Médicaments qui contiennent un des composés conforme aux révindications 1 à 3
